# EUROPÄISCHE PATENTSCHRIFT

(11) **EP 3 148 398 B1**
(45) Veröffentlichungstag und Bekanntmachung des Hinweises auf die Patenterteilung: **02.01.2019**
(21) Anmeldenummer: 16732534.9
(22) Anmeldetag: 13.06.2016
(51) Int. Cl.: A61B 1/015, B65D 30/24, B65D 83/00, B65D 47/04, B65D 47/20, B65D 47/24, A61M 1/00

(54) **SEPARATE AUFNAHMEEINRICHTUNG MIT INTEGRIERTER VENTILVORRICHTUNG FÜR VER- UND/ODER ENTSORGUNGSBEHÄLTER ZUR REINIGUNG DER BETRIEBSMASCHINE EINES ENDOSKOPS**
SEPARATE RECEIVING DEVICE WITH INTEGRATED VALVE DEVICE FOR SUPPLY AND/OR DISPOSAL VESSELS FOR THE CLEANING OF THE OPERATING MACHINE OF AN ENDOSCOPE
DISPOSITIF DE RÉCEPTION SÉPARÉ COMPRENANT UN DISPOSITIF DE VANNE INTÉGRÉ POUR RÉCIPIENTS D'ALIMENTATION ET/OU D'ÉVACUATION POUR LE NETTOYAGE DE LA MACHINE D'EXPLOITATION D'UN ENDOSCOPE

(30) Priorität: 14.08.2015 DE 102015113429
(43) Veröffentlichungstag der Anmeldung: 05.04.2017
(73) Patentinhaber: Ambu A/S, 2750 Ballerup (DK)
(72) Erfinder: FIEBER, Markus Dr., 80636 München (DE); ABICHT, Felix, 82337 Wörthsee (DE); HOMANN, Till, 82278 Althegnenberg (DE); ILG, Dylan, 82266 Inning (DE); POMMERSHEIM, Wilhelm, 86477 Adelsried (DE); AFHOLDERBACH, Tim, 86165 Augsburg (DE); VON RUEPPRECHT, Oliver, 86343 Königsbrunn (DE)
(74) Vertreter: Winter, Brandl, Fürniss, Hübner, Röss, Kaiser, Polte - Partnerschaft mbB
(86) Internationale Anmeldenummer: PCT/EP2016/063489
(87) Internationale Veröffentlichungsnummer: WO 2017/028979

(56) Entgegenhaltungen:
- JP-A- 2012 130 647
- US-A- 5 447 237
- US-A1- 2003 168 500
- US-A1- 2007 043 262

## Beschreibung

Die vorliegende Erfindung betrifft eine Aufnahmeeinrichtung bzw. ein Reinigungskit oder -einheit zur Aufnahme von einem oder mehreren Ver- und/oder Entsorgungsbehältern zur Reinigung der Betriebsmaschine eines Endoskops gemäß dem Oberbegriff des Anspruchs 1.

### Hintergrund der Erfindung

Endoskope sind medizinische Arbeitsgerätschaften, mit denen das Innere von in der Regel lebenden Organismen untersucht und/oder manipuliert werden kann. Ursprünglich für die humanmedizinische Diagnostik entwickelt, werden sie heute auch für minimal-invasive operative Eingriffe an Mensch und Tier eingesetzt. Zu den Endoskopen zählen sowohl starre als auch flexible Endoskope und deren Unterarten.

Endoskope weisen in der Regel am distalen Ende des starren Endoskopschafts bzw. flexiblen Endoskopschlauchs einen Endoskopkopf auf, der eine Optik oder andere Bild- oder Videoeinrichtungen sowie ein Leuchtmittel zum Beleuchten des Untersuchungs- oder Operationsfelds aufweist. Über Arbeitskanäle können durch den Endoskopschaft bzw. -schlauch hindurch mikromechanische Geräte oder Instrumente wie kleine Zangen oder Greifer oder Scheren bis zum Operationsfeld eingeführt werden. Ferner kann der Endoskopkopf auch elektrochirurgische Einrichtungen, z. B. zum Koagulieren oder Fusionieren von Gewebe oder Hohlgefäßen, aufweisen. Über die Arbeitskanäle im Inneren des Endoskops werden auch Versorgungs- und/oder Betriebsmittel, wie Spülflüssigkeiten, eingebracht und auch Patientengewebe und/oder Körperflüssigkeiten, wie Blut oder Sekret, aber auch die eingebrachten Versorgungs- und/oder Betriebsmittel wieder abgesaugt bzw. abgeführt. Beispielsweise muss während der Operation die Optik des Endoskopkopfes gereinigt werden, so dass der Chirurg über das Okular oder elektronische Bildwiedergabeeinheit das Operationsfeld einsehen kann.

Die Versorgung des Endoskops mit diesen Flüssigkeiten sowie Energie, aber auch die Entsorgung von Gewebe oder Flüssigkeiten aus dem Endoskop erfolgt über eine Betriebsmaschine, mit welcher das Endoskop über entsprechende Leitungen gekoppelt werden kann. In der Betriebsmaschine sind auch die Pumpen angeordnet, mittels welchen die Mittel zu- und abgeführt werden können. Diese Flüssigkeiten werden aus und in entsprechende Tanks, welche in der Betriebsmaschine angeordnet sind, über entsprechend integrierte Leitungen gefördert.

Aufgrund des Austausches von Flüssigkeiten zwischen Betriebsmaschine und Patienten besteht die Gefahr, dass sich Patienten, welche mit derselben Betriebsmaschine behandelt werden, oder auch das Klinikpersonal infizieren können, weshalb an solche Systeme höchste Reinheitsanforderungen gestellt werden. Durch die Integration des Ver- und/oder Entsorgungssystems in der Betriebsmaschine lassen sich diese Hygieneanforderungen oftmals nur schwer oder mit erheblichem Aufwand einhalten. Beispielsweise müssen die Ver- und/oder Entsorgungsbehälter und auch die Zu- und Ablaufleitungen desinfiziert oder sterilisiert werden und hierfür aus der Betriebsmaschine ausgebaut werden.

Die US 2007/0043262 A1 offenbart beispielsweise eine Vorrichtung für ein Endoskop mit einer Flüssigkeitsversorgungseinheit. Die Flüssigkeitsversorgungseinheit weist mehrere nachfüllbare und austauschbare Flüssigkeitsbehälter auf, welche einem Endoskop, mit Hilfe eines mit den Flüssigkeitsbehältern verbunden Luftdrucksystems, Flüssigkeit zuführen können. Hierbei ist jeder Flüssigkeitsbehälter gegenüber der Umgebung geschlossen und weist nur einen Eingang und einen Ausgang auf, wobei an dem Eingang Luft für einen Überdruck eingegeben wird und an dem Ausgang das Fluid durch den Überdruck hinausgepresst wird.

Vor diesem Hintergrund besteht die Aufgabe der vorliegenden Erfindung darin, für die Reinigung der Betriebsmaschine ein Ver- und/oder Entsorgungssystem bereitzustellen, welches auf einfache Weise höheren Krankenhaushygieneanforderungen genügt.

Diese Aufgabe wird durch eine Aufnahmeeinrichtung mit den Merkmalen des Anspruchs 1 gelöst. Vorteilhafte Weiterbildungen sind Gegenstand von Unteransprüchen.

Erfindungsgemäß wird eine Aufnahmeeinrichtung zur Aufnahme von einem oder mehreren Ver- und/oder Entsorgungsbehältern bereitgestellt, von welchen Versorgungs- und/oder Betriebsmittel, wie Desinfektionsmittel, Alkohol oder andere Spülflüssigkeiten zur Betriebsmaschine gefördert werden und nach dem Durchlauf durch die hydraulischen Systeme der Betriebsmaschine zu Reinigungszwecken wieder der Aufnahmeeinrichtung zurückgeführt werden.

Der Kerngedanke der Erfindung liegt darin, dass die Aufnahmeeinrichtung separat zu der Betriebsmaschine ausgebildet ist und eine integrierte Ventilvorrichtung in Form einer integrierten Steuerventilleiste mit mehreren Ventilen aufweist, mittels welcher Volumenströme in Ver- und/oder Entsorgungsleitungen, welche einerseits mit den Ver- und/oder Entsorgungsbehältern und andererseits mit der Pump- und/oder Saugvorrichtung in der Betriebsmaschine koppelbar sind, gesteuert werden können.

Somit bildet die erfindungsgemäße Aufnahmeeinrichtung eine von der Betriebsmaschine separate und unabhängige Reinigungseinheit, die unabhängig von der eigentlichen Endoskopie vorbereitet, z.B. durch Auffüllen oder Bestücken mit Betriebsmitteln und wieder aufbereitet werden kann. Diese kann daher mit Einwegbehältern oder auch Mehrfachbehältern ausgestattet werden. Da die Aufnahmeeinrichtung auch mehrere Ver- und/oder Entsorgungsbehälter aufnehmen kann, wird dadurch auch die Handhabung vereinfacht, so dass nicht einzelne Behälter aus der Betriebsmaschine entnommen werden müssen und einzeln zum Sterilisieren gebracht werden müssen, sondern dass diese zusammen in der Aufnahmeeinrichtung zum Desinfizieren gebracht werden können.

Gemäß einem Aspekt kann eine separate, in die Aufnahmeeinrichtung integrierte Steuereinrichtung über eine Steuerleitung zwischen Betriebsmaschine und Aufnahmeeinrichtung von einer in der Betriebsmaschine angeordneten Steuereinrichtung angesteuert werden und für den anschließenden Reinigungsvorgang die Masterfunktion übernehmen. Durch die ausgelagerte Steuereinrichtung und Ventilvorrichtung, welche in der Aufnahmeeinrichtung integriert ist, kann das Ver- und/oder Entsorgungssystem auf einfache Weise um bestimmte Behälter ergänzt oder reduziert werden und so unterschiedliche Reinigungsprozeduren (z.B. High-Level-Desinfektion oder Low-Level-Desinfektion) einfach realisiert werden. Die Steuerung der Ventilvorrichtung kann über eine pneumatische, hydraulische oder elektrische Steuerleitung aus erfolgen.

Gemäß einem Aspekt kann die Ventilvorrichtung für jeden Ver- und/oder Entsorgungsbehälter ein separates und individuell ansteuerbares Ventil aufweisen. Dadurch lässt sich die Versorgung mit den einzelnen Versorgungs- und/oder Betriebsmitteln individuell und bedarfsgerecht einstellen.

Gemäß einem Aspekt der Erfindung kann die Ventilvorrichtung Schlauchquetschventile aufweisen. Der Vorteil solcher Schlauchquetschventile liegt in der Reinigung, da die Ventile selbst nicht mit den Flüssigkeiten in Kontakt kommen und die einzelnen Schläuche sehr einfach aus der Ventileinrichtung entnommen und durch neue oder sterilisierte Schläuche ersetzt werden können.

Gemäß einem Aspekt können die von den Versorgungsbehältern jeweils wegführenden Versorgungsleitungen nach der Ventilvorrichtung bzw. nach den jeweiligen Ventilen in einer gemeinsamen Versorgungsleitung münden. Die einzelnen Flüssigkeiten, wie ortho-Phthaldialdehyd (OPA) oder Isopropylalkohol (ISO) oder H₂O etc. werden über einen gemeinsamen Kanal der Betriebsmaschine in frei definierbaren Sequenzen zugeführt. Vor diesem Hintergrund müssen nicht separate Pumpleitungen und Pumpen vorgesehen werden. Menge und Art des Versorgungsmittels können durch entsprechende Betätigung der Ventile in der Ventilvorrichtung ausgewählt bzw. gesteuert werden. Darüber hinaus steuert das Programm der Aufnahmeeinheit auch die entsprechenden Pumpen und Ventile in der Betriebsmaschine.

Gemäß einem Aspekt können die Versorgungsleitung(en) und/oder die Entsorgungsleitung(en) und/oder die Steuerleitung(en) eine gemeinsame Schnittstelle mit der Betriebsmaschine aufweisen. Somit kann zur weiteren Vereinfachung der Handhabung der Aufnahmeeinrichtung und der Leitungen zwischen der Aufnahmeeinrichtung und der Betriebsmaschine die Schnittstellen mittels einer Multifunktionseinheit reduziert werden. Somit können zwei oder mehrere Leitungen über eine Multifunktionseinheit mit der Betriebsmaschine gekoppelt werden.

Gemäß einem Aspekt der Erfindung kann die Aufnahmeeinrichtung Befestigungsmittel zur lösbaren Befestigung der Aufnahmeeinrichtung an der Betriebsmaschine aufweisen. Beispielsweise kann die Aufnahmeeinrichtung einen oder mehrere Haken aufweisen, die in entsprechende Halterungen oder Aufnahmen an der Betriebsmaschine eingehängt werden können, so dass die Aufnahmeeinrichtung werkzeuglos an der Betriebsmaschine befestigt oder von dieser entnommen werden kann. Dies erleichtert beispielsweise die Handhabung, um die Aufnahmeeinrichtung nicht separat tragen zu müssen, wenn die Betriebsmaschine von einem Raum in einen anderen Raum gebracht wird.

Gemäß einem Aspekt kann die Aufnahmeeinrichtung in Form eines offenen Trägers mit definierten Behälterstellplätzen ausgebildet sein. Auf diese Weise können die einzelnen Behälter sehr einfach entnommen oder in den Träger eingesetzt werden. Durch die definierten Behälterstellplätze kann eine vorbestimmte Anordnung der Behälter z.B. in Bezug auf die Steuerventile vorgegeben werden. Wenn sich die definierten Behälterstellplätze in Größe und Form unterscheiden, kann dadurch auch sichergestellt werden, dass nur bestimmte Behälter in bestimmte Behälterstellplätze eingesetzt werden können und es so zu keinen Verwechselungen zwischen den unterschiedlichen Versorgungsmitteln kommt.

Gemäß einem Aspekt der Erfindung können die Behälterstellplätze den Ver- und/oder Entsorgungsbehältern entsprechend gekennzeichnet oder beschriftet sein. So kann beispielsweise der Behälterstellplatz für den H₂O-Versorgungsbehälter entsprechend die Kennzeichnung "H₂O" oder Farbcodierung aufweisen, damit die Ventilvorrichtung und die entsprechende Steuervorrichtung nicht jedes Mal neu auf die einzelnen Ver- und Entsorgungsbehälter angepasst und umprogrammiert werden müssen.

Gemäß einem Aspekt kann die Aufnahmeeinrichtung mittig und oberhalb der Ver- und/oder Entsorgungsbehälter einen, insbesondere ergonomisch ausgebildeten, Handgriff aufweisen. Durch den Handgriff wird die Handhabung der Aufnahmeeinrichtung vor und nach einer Reinigung der Betriebsmaschine erleichtert.

Dieser Handgriffabschnitt oder auch ein anderer oberhalb der Stellplätze vorgesehener Abschnitt des Trägers oder der Aufnahmeeinrichtung kann auch die erfindungsgemäße integrierte Steuerventilleiste aufweisen. Der Handgriff samt Steuerventilleiste kann zum Einsetzen und/oder Entnehmen der Ver- und/oder Entsorgungsbehälter zur Seite wegklappbar sein. Mittels der oberhalb der Stellplätze angeordneten Steuerventilleiste ist das jeweilige Ventil unmittelbar oberhalb des jeweiligen Ver- und/oder Entsorgungsbehälters angeordnet. Wenn diese Steuerventilleiste wegklappbar ist, kann die Aufnahmeeinrichtung leichter mit den entsprechenden Ver- und/oder Entsorgungsbehältern bestückt werden.

Die vorliegende Erfindung wird anhand einer bevorzugten Ausführungsform mit Bezug auf die beigefügten Figuren nachfolgend detaillierter dargestellt.

### Kurzbeschreibung der Zeichnungen

Fig. 1 zeigt ein Endoskop, eine Betriebsmaschine des Endoskops sowie eine Aufnahmeeinrichtung gemäß einer bevorzugten Ausführungsform der Erfindung; und
Fig. 2 zeigt eine Detailansicht der Aufnahmeeinrichtung gemäß der bevorzugten Ausführungsform der Erfindung

### Detaillierte Beschreibung der bevorzugten Ausführungsform

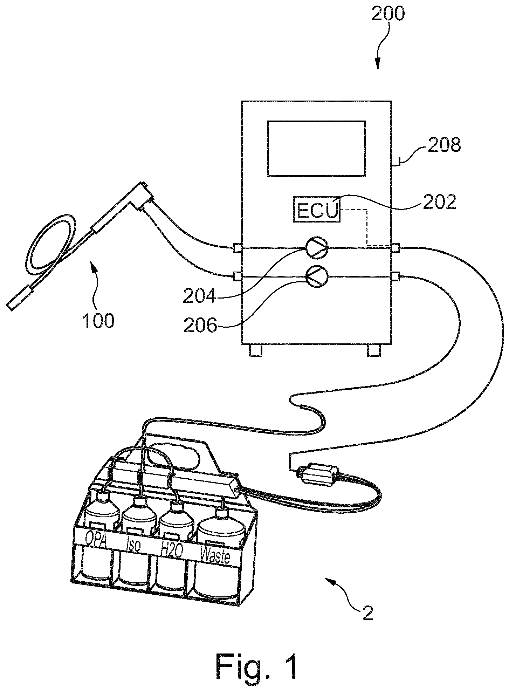

Fig.1 zeigt Endoskopiesystem mit einem Endoskop 100, das über diverse Leitungen (u.a. Ver- und Entsorgungsleitungen) mit einer Betriebsmaschine 200 des Endoskops 100 gekoppelt ist, und einer ebenfalls mit der Betriebsmaschine 200 (über weiter unten im Detail beschriebene Leitungen) gekoppelten erfindungsgemäßen Aufnahmeeinrichtung 2.

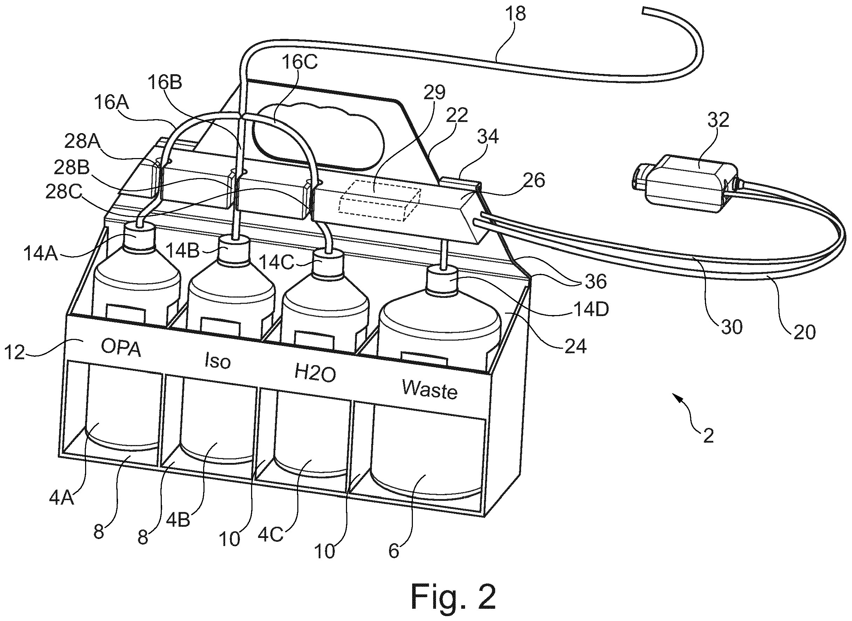

Fig. 2 zeigt eine Detailansicht einer erfindungsgemäßen Aufnahmeeinrichtung 2 in Form eines Trägers. In dem Träger 2 sind drei Versorgungsbehälter 4 und ein Entsorgungsbehälter 6 aufgenommen. Der Träger 2 weist hierfür entsprechende Stellplätze 8 auf, welche durch Trennwände 10 voneinander in einzelne Abteile unterteilt sind. Die Stellplätze 8 können sich -wie dies auch bei der in Fig. 1 dargestellten Ausführungsform der Fall ist - in ihrer Größe unterscheiden, um unterschiedliche Größen von Ver- und/oder Entsorgungsbehältern 4 und 6 aufnehmen zu können. Die Behälter 4 und 6 sind lose in dem Träger 2 bzw. die Stellplätze 8 gestellt und können jederzeit entnommen und durch andere ersetzt werden. Die einzelnen Stellplätze 8 sind auf einem nach vorne weisenden Seitenwandteil 12 entsprechend markiert bzw. beschriftet. Im vorliegenden Fall ist der Träger 2 dafür vorgesehen, als Versorgungsbehälter 4 einen Behälter mit OPA, einen Behälter mit Isopropylalkohol und einen Behälter mit H₂O sowie einen Absaug- oder Abfallbehälter 6 aufzunehmen. Die Versorgungsbehälter 4 und der Entsorgungsbehälter 6 sind an ihrer Oberseite jeweils über einen Verschluss 14 verschlossen.

Die Versorgungsbehälter 4A, 4B und 4C sind über entsprechende Zweigversorgungsleitungen 16A, 16B und 16C mit einer gemeinsamen Versorgungsleitung 18 verbunden. Die Zweigversorgungsleitungen 16A, 16B, 16C sind mit den jeweiligen Verschlüssen 14A, 14B und 14C der Versorgungsbehälter 4A, 4B und 4C verbunden und ermöglichen somit das Abpumpen der entsprechenden Versorgungsmittel aus den Versorgungsbehältern 4A, 4B und 4C über die Betriebsmaschine 200. Zum Verschluss 14D des Entsorgungsbehälters 6 ist eine Entsorgungsleitung 20 geführt, welche von der Betriebsmaschine 200 abgesaugte Flüssigkeiten in den Entsorgungsbehälter 6 abtransportiert.

Unmittelbar oberhalb der Stellplätze 8 befindet sich ein Griffabschnitt 22, der mit einer Rückwand 24 des Trägers 2 verbunden ist. Am Griffabschnitt 22 oder unmittelbar unterhalb davon ist eine Steuerventilleiste 26 vorgesehen, welche mehrere Ventile zur Steuerung der Volumenströme in den Zweigversorgungsleitungen 16A, 16B, 16C bzw. in der gemeinsamen Versorgungsleitung 18 aufweist. Genauer gesagt sind drei Schlauchquetschventile 28A, 28B und 28C vorgesehen, in welche die Zweigversorgungsleitungen 16A, 16B und 16C eingesetzt sind und über welche die durch die Zweigversorgungsleitungen 16A, 16B und 16C geführten Volumenströme gesteuert werden können.

Eine in der Steuerventilleiste 26 integrierte Steuereinrichtung oder -element 29 ist über eine Steuerleitung 30 mit der in der Betriebsmaschine 200 angeordneten Steuereinrichtung 202 (siehe Fig. 1) verbunden und steuert sowohl die einzelnen Schlauchquetschventile 28A, 28B und 28C als auch die entsprechenden Pumpen 204, 206 und Ventile in der Betriebsmaschine 200 während des Reinigungsvorganges. Die Ansteuerung der Ventile 28A, 28B und 28C kann pneumatisch, hydraulisch oder elektrisch erfolgen.

Der durch eine in der Betriebsmaschine 200 angeordneten (Versorgungs-)Pumpe 204 erzeugte Unterdruck führt dazu, dass bei Öffnen der Schlauchquetschventile 28 die entsprechenden Versorgungsmittel in den Versorgungsbehältern 4 über die Versorgungsleitungen 16 und 18 zur Betriebsmaschine 200 gepumpt bzw. gefördert werden. In umgekehrter Richtung werden Flüssigkeiten aus der Betriebsmaschine 200 mittels einer (Entsorgungs-)Pumpe 206 über die Entsorgungsleitung 20 in den Entsorgungsbehälter 6 abgesaugt.

Gemäß der vorliegenden Ausführungsform ist für die Entsorgungsleitung 20 kein Ventil in der Steuerventilleiste 26 vorgesehen. Es ist jedoch auch denkbar, den Volumenstrom der Entsorgungsleitung 20 zu steuern oder kurzzeitig zu unterbinden, z.B. beim Wechseln des Entsorgungsbehälters 6.

Wie aus der Fig. 1 zu erkennen ist, sind die Entsorgungsleitung 20 und die Steuerleitung 30 über eine gemeinsame Schnittstelle bzw. eine Multifunktionseinheit 32 mit der Betriebsmaschine 200 koppelbar. In einer Variante kann auch die Versorgungsleitung 18 mit der Steuerleitung 30 oder auch die Versorgungsleitung 18 und Entsorgungsleitung 20 oder auch alle drei Leitungen 18, 20 und 30 kombiniert werden, um so die Handhabung durch Reduzierung der Schnittstellen mit der Betriebsmaschine 200 zu vereinfachen.

Der Träger 2 weist auf der Rückseite des Griffabschnitts 22 zwei oder mehrere hakenförmige Leisten 34 auf, mittels welchen der Träger 2 an einer Halterung 208 neben oder an der Betriebsmaschine 200 eingehängt werden kann.

Ferner ist der Griffabschnitt 22 über zwei Filmscharniere 36 an der Rückwand 24 des Trägers 2 angebunden, so dass zum Einsetzen oder Entnehmen der Behälter 4 und 6 der Griffabschnitt 22 samt Steuerventilleiste 26 zur (Rück-)Seite hin weggeklappt werden können, um so die Zugänglichkeit der Stellplätze 8 von oben zu verbessern bzw. zu erleichtern.

Im Einsatz bilden der Träger 2 einschließlich der Ver- und Entsorgungsleitungen 18 und 20 sowie Steuerleitung 30 eine separate Einheit oder ein separates Modul, das auf sehr einfache Weise mit der Betriebsmaschine 200 gekoppelt und von dieser entkoppelt werden kann. Auf diese Weise können die Ver- und Entsorgungsleitungen 18 und 20 sehr einfach entsorgt oder desinfiziert werden. Gleiches gilt für die Versorgungs- und Entsorgungsbehälter 4 und 6. Auch der Träger 2 kann als Ein- oder Mehrwegprodukt konzipiert sein. Aufgrund seines Aufbaus lässt sich auch dieser sehr leicht desinfizieren. Durch den modularen Aufbau des Trägers 2 und den drei definierten Schnittstellen (Versorgungsleitung 18, Entsorgungsleitung 20 und Steuerleitung 30) können auch unterschiedlich vorkonfektionierte Träger 2 mit unterschiedlichen Ver- und Entsorgungsbehältern 4 und 6 an der Betriebsmaschine 200 ohne großen Aufwand und je nach Bedarf angekoppelt werden.

Die vorliegende Erfindung ist jedoch nicht auf die beschriebene Ausführungsform beschränkt und kann auf verschiedene Weisen abgewandelt werden. Beispielsweise könnte der Träger 2 zwei Reihen von Stellplätzen 8 mit unterschiedlich konfektionierten Behältern 4 und 6 aufweisen, um durch Austauschen der Versorgungsleitungen 16 in den Schlauchquetschventilen 28 mit ein und demselben Träger 2 unterschiedliche Versorgungsbehälter 4 und 6 wahlweise zu steuern. Der Griffabschnitt 22 samt Steuerventilleiste 26 kann nicht nur wie bei der bevorzugten Ausführungsform einstückig mit dem Träger 2 verbunden sein, sondern auch mit dem Gefache für die Behälter 4 und 6 loslösbar verbunden sein, so dass dieser mit anderen Trägern 2 verbindbar ist.

### Bezugszeichenliste

- 2: Träger
- 4: Versorgungsbehälter
- 6: Entsorgungsbehälter
- 8: Stellplatz
- 10: Trennwand
- 12: Seitenwandteil
- 14: Verschluss
- 16: Zweigversorgungsleitung
- 18: Versorgungsleitung
- 20: Entsorgungsleitung
- 22: Griffabschnitt
- 24: Rückwand
- 26: Steuerventilleiste
- 28: Schlauchquetschventil
- 29: Steuereinrichtung
- 30: Steuerleitung
- 32: Multifunktionseinheit
- 34: Leisten
- 36: Filmscharniere
- 100: Endoskop
- 200: Betriebsmaschine
- 202: Steuereinrichtung (ECU)
- 204: Pumpe
- 206: Pumpe
- 208: Halterung

## Patentansprüche

1. Aufnahmeeinrichtung (2) zur Aufnahme von einen oder mehreren Ver- und/oder Entsorgungsbehältern (4, 6), von welchen Versorgungs- und/oder Betriebsmittel, wie Desinfektionsmittel oder Spülflüssigkeiten, oder zu welchen Körperflüssigkeiten, wie Blut oder Sekret, über eine in einer Betriebsmaschine (200) des Endoskops (100) angeordnete Pump- und/oder Saugvorrichtung (204, 206) gefördert werden können, wobei die Aufnahmeeinrichtung (2) separat zu der Betriebsmaschine (200) ausgebildet ist und **dadurch gekennzeichnet, dass** die Aufnahmeeinrichtung (2) eine integrierte Ventilvorrichtung (26, 28) in Form einer integrierten Steuerventilleiste (26) mit mehreren Ventilen (28) aufweist, mittels welcher Volumenströme der Versorgungsmittel und/oder Betriebsmittel in Versorgungsleitungen (16, 18) und/oder der Körperflüssigkeiten in Entsorgungsleitungen (20), welche einerseits mit den Ver- und/oder Entsorgungsbehältern (4, 6) und andererseits mit der Pump- und/oder Saugvorrichtung (204, 206) in der Betriebsmaschine (200) koppelbar sind, gesteuert werden können.

2. Aufnahmeeinrichtung (2) nach Anspruch 1, ferner **gekennzeichnet durch** eine, vorzugsweise in die Aufnahmeeinrichtung integrierte, Steuereinrichtung (29), über welche die Ventilvorrichtung (26, 28) in der Aufnahmeeinrichtung (2) angesteuert werden kann und/oder über welche über eine zwischen der Aufnahmeeinrichtung (2) und der Betriebsmaschine (200) anordbare, insbesondere pneumatische, Steuerleitung (30) in der Betriebsmaschine (200) angeordnete Pumpen (204, 206) und/oder Ventile angesteuert werden können.

3. Aufnahmeeinrichtung (2) nach Anspruch 1 oder 2, **dadurch gekennzeichnet, dass** die Ventilvorrichtung (26, 28) für jeden Ver- und/oder Entsorgungsbehälter (4, 6) ein separates und individuell ansteuerbares Ventil (28), insbesondere ein Schlauchquetschventil (28), vorzugsweise ein elektro-mechanisch ansteuerbares Schlauchquetschventil (28), aufweist.

4. Aufnahmeeinrichtung (2) nach Anspruch 3, **dadurch gekennzeichnet, dass** die Ver- und/oder Entsorgungsleitungen (16, 18, 20) werkzeuglos in die Schlauchquetschventile (28) eingesetzt und aus diesen herausgenommen werden können.

5. Aufnahmeeinrichtung (2) nach einem der vorhergehenden Ansprüche, **dadurch gekennzeichnet, dass** von den Versorgungsbehältern (4) jeweils wegführende Versorgungsleitungen (16) nach der Ventilvorrichtung (26, 28) in einer gemeinsamen Versorgungsleitung (18) münden.

6. Aufnahmeeinrichtung (2) nach einem der vorhergehenden Ansprüche, **dadurch gekennzeichnet, dass** zwei oder mehrere aus den Ver- und/oder Entsorgungsleitungen (16, 18, 20) und der Steuerleitung (30) eine gemeinsame Schnittstelle mit der Betriebsmaschine (200), vorzugsweise in Form einer Multifunktionseinheit (32), aufweisen.

7. Aufnahmeeinrichtung (2) nach einem der vorhergehenden Ansprüche, **dadurch gekennzeichnet, dass** die Aufnahmeeinrichtung mittig und oberhalb der Ver- und/oder Entsorgungsbehältern (4, 6) einen, insbesondere ergonomisch ausgebildeten, Handgriff (22) mit der integrierten Steuerventilleiste (26) aufweist, wobei der Handgriff (22) samt der integrierten Steuerventilleiste (26) zum Einsetzen oder Entnehmen der Ver- und/oder Entsorgungsbehältern (4, 6) zur Seite wegklappbar ist.

8. Aufnahmeeinrichtung (2) nach einem der vorhergehenden Ansprüche, **dadurch gekennzeichnet, dass** die Aufnahmeeinrichtung Befestigungsmittel (34), insbesondere Haken, zum lösbaren Befestigen der Aufnahmeeinrichtung (2) an der Betriebsmaschine (200) aufweist.

9. Aufnahmeeinrichtung (2) nach einem der vorhergehenden Ansprüche, **dadurch gekennzeichnet, dass** die Aufnahmeeinrichtung (2) in Form eines offenen Trägers mit definierten, insbesondere unterschiedliche großen, Behälterstellplätzen (8) ist, welche vorzugsweise den Ver- und/oder Entsorgungsbehältern (4, 6) entsprechend gekennzeichnet oder beschriftet sind.

## Claims

1. Receiving device (2) for the receipt of one or more supply containers and/or disposal containers (4, 6), from which supply means and/or operating means such as disinfection means or rinsing fluids, or to which bodily fluids such as blood or secretion, can be conveyed via a pump device and/or suction device (204, 206) disposed in an operating machine (200) of the endoscope (100),
wherein
the receiving device (2) is designed separate from the operating machine (200) and **characterised in that**
the receiving device (2) has an integrated valve device (26, 28) in the form of an integrated control valve bar (26) with a plurality of valves (28) by means of which volume flows of the supply means and/or operating means into supply lines (16, 18) and/or of bodily fluids into disposal lines (20), which can be coupled on the one hand with the supply containers and/or disposal containers (4, 6) and on the other hand with the pump device and/or suction device (204, 206) in the operating machine (200), can be controlled.

2. Receiving device (2) according to claim 1, further characterised through a, preferably integrated into the receiving device, control device (29) via which the valve device (26, 28) in the receiving device (2) can be controlled and/or via which pumps (204, 206) and/or valves disposed in the operating machine (200) can be controlled via an, in particular pneumatic, control line (30) which can be disposed between the receiving device (2) and the operating machine (200).

3. Receiving device (2) according to claim 1 or 2, **characterised in that** the valve device (26, 28) has for each supply container and/or disposal container (4, 6) a separate and individually controllable valve (28), in particular a tubing pinch valve (28), preferably an electro-mechanically controllable tubing pinch valve (28).

4. Receiving device (2) according to claim 3, **characterised in that** the supply lines and or disposal lines (16, 18, 20) can without tools be fitted into the tubing pinch valves (28) and removed therefrom.

5. Receiving device (2) according to any of the preceding claims, **characterised in that** supply lines (16) leading away respectively from the supply containers (4), after the valve device (26, 28) open out in a shared supply line (18).

6. Receiving device (2) according to any of the preceding claims, **characterised in that** two or more of the supply lines and/or disposal lines (16, 18, 20) and the control line (30) have a shared interface with the operating machine (200), preferably in the form of a multifunction unit (32).

7. Receiving device (2) according to any of the preceding claims, **characterised in that** the receiving device has centrally and above the supply containers and or disposal containers (4, 6) an, in particular ergonomically designed, handle (22) with the integrated control valve bar (26), wherein the handle (22) together with the integrated control valve bar (26) can be folded away to the side for inserting or removing the supply containers and or disposal containers (4, 6).

8. Receiving device (2) according to any of the preceding claims, **characterised in that** the receiving device comprises fastening means (34), in particular hooks, for the releasable fastening of the receiving device (2) to the operating machine (200).

9. Receiving device (2) according to any of the preceding claims, **characterised in that** the receiving device (2) is in the form of an open carrier with defined, in particular differently sized container standing places (8), which are preferably marked or labelled corresponding to the supply containers or disposal containers (4, 6).

## Revendications

1. Dispositif de logement (2) destiné au logement d'un ou plusieurs récipients d'approvisionnement et/ou d'élimination (4, 6), à partir desquels des produits d'approvisionnement et/ou de fonctionnement, tels que des produits désinfectants ou des liquides de rinçage, ou vers lesquels des liquides corporels, tels que du sang ou des sécrétions, peuvent être transportés par l'intermédiaire d'un dispositif de pompage et/ou d'aspiration (204, 206) agencé dans une machine opératrice (200) de l'endoscope (100),
lequel dispositif de logement (2) est réalisé séparé de la machine opératrice (200),
**caractérisé en ce que** le dispositif de logement (2) comporte un dispositif de vannes (26, 28) intégré, sous la forme d'une barre de vannes de commande (26) intégrée comportant plusieurs vannes (28), au moyen duquel peuvent être commandés des flux des produits d'approvisionnement et/ou des produits de fonctionnement dans des conduits d'approvisionnement (16, 18) et/ou des liquides corporels dans des conduits d'élimination (20), lesquels conduits peuvent être couplés d'une part aux récipients d'approvisionnement et/ou d'élimination (4, 6) et d'autre part au dispositif de pompage et/ou d'aspiration (204, 206) dans la machine opératrice (200).

2. Dispositif de logement (2) selon la revendication 1, **caractérisé en outre par** un dispositif de commande (29), qui est intégré de préférence dans le dispositif de logement et par l'intermédiaire duquel le dispositif de vannes (26, 28) dans le dispositif de logement (2) peut être commandé et/ou par l'intermédiaire duquel des pompes (204, 206) et/ou vannes agencées dans la machine opératrice (200) peuvent être commandées par l'intermédiaire d'une conduite de commande (30), en particulier pneumatique, pouvant être agencée entre le dispositif de logement (2) et la machine opératrice (200).

3. Dispositif de logement (2) selon la revendication 1 ou 2, **caractérisé en ce que** le dispositif de vannes (26, 28) comporte pour chaque récipient d'approvisionnement et/ou d'élimination (4, 6) une vanne (28) séparée et pouvant être commandée individuellement, en particulier une vanne à manchon déformable (28), de préférence une vanne à manchon déformable (28) à commande électromécanique.

4. Dispositif de logement (2) selon la revendication 3, **caractérisé en ce que** les conduits d'approvisionnement et/ou d'élimination (16, 18, 20) peuvent être placés sans outil dans les vannes à manchon déformable (28) et retirés de ceux-ci de même.

5. Dispositif de logement (2) selon l'une quelconque des revendications précédentes, **caractérisé en ce que** des conduits d'approvisionnement (16) éloignant à chaque fois des récipients d'approvisionnement (4) débouchent dans un conduit d'approvisionnement (18) commun après le dispositif de vannes (26, 28).

6. Dispositif de logement (2) selon l'une quelconque des revendications précédentes, **caractérisé en ce que** deux ou plusieurs des conduits d'approvisionnement et/ou d'élimination (16, 18, 20) et de la conduite de commande (30) comportent une interface commune avec la machine opératrice (200), de préférence sous la forme d'une unité multifonctionnelle (32).

7. Dispositif de logement (2) selon l'une quelconque des revendications précédentes, **caractérisé en ce que** le dispositif de logement comporte, au centre et au-dessus des récipients d'approvisionnement et/ou d'élimination (4, 6), une poignée (22) qui est réalisée en particulier de façon ergonomique et qui comprend la barre de vannes de commande (26) intégrée, laquelle poignée (22) peut être basculée de côté, avec la barre de vannes de commande (26) intégrée, afin de mettre en place ou de retirer les récipients d'approvisionnement et/ou d'élimination (4, 6).

8. Dispositif de logement (2) selon l'une quelconque des revendications précédentes, **caractérisé en ce que** le dispositif de logement dispose des moyens de fixation (34), en particulier des crochets, afin de fixer de manière amovible le dispositif de logement (2) sur la machine opératrice (200).

9. Dispositif de logement (2) selon l'une quelconque des revendications précédentes, **caractérisé en ce que** le dispositif de logement (2) est sous la forme d'un support ouvert avec des emplacements de récipients (8) définis, en particulier de dimensions différentes, qui sont marqués ou écrits de préférence de manière correspondante aux récipients d'approvisionnement et/ou d'élimination (4, 6).
